# EUROPEAN PATENT SPECIFICATION

(11) **EP 3 451 953 B1**
(45) Date of publication and mention of the grant of the patent: **10.12.2025**
(21) Application number: 17793452.8
(22) Date of filing: 05.05.2017
(51) Int. Cl.: A61B 17/3211, A61B 17/22, A61B 17/3207, A61M 25/10, A61B 17/32, A61B 17/00, A61F 2/01

(54) **INTERNAL CAROTID ARTERY THROMBECTOMY DEVICES**
VORRICHTUNGEN ZUR THROMBEKTOMIE DER INNEREN HALSSCHLAGADER
DISPOSITIFS DE THROMBECTOMIE DE L'ARTÈRE CAROTIDE INTERNE

(30) Priority: 06.05.2016 US 201662332495 P
(43) Date of publication of application: 13.03.2019
(73) Proprietor: Mayo Foundation for Medical Education and Research, Rochester, MN 55905 (US)
(72) Inventor: BRINJIKJI, Waleed, Rochester, MN 55902 (US); KALLMES, David, F., Rochester, MN 55902 (US)
(74) Representative: Gray, James
(86) International application number: PCT/US2017/031311
(87) International publication number: WO 2017/192999

(56) References cited:
- WO-A1-01/91844
- WO-A1-2014/204860
- WO-A2-03/061457
- WO-A2-2010/017537
- US-A- 5 135 482
- US-A- 5 263 959
- US-A- 5 897 537
- US-A1- 2003 023 204
- US-A1- 2006 198 976
- US-A1- 2007 270 740
- US-A1- 2009 306 597
- US-A1- 2012 116 350
- US-A1- 2014 107 575
- US-A1- 2014 107 575

## Description

### CROSS REFERENCE TO RELATED APPLICATION

This application claims the benefit of U.S. Provisional Application Serial No. 62/332,495 filed on May 6, 2016 and entitled System and Method for Interventional Cardiac Device.

### FIELD OF THE INVENTION

The disclosure relates generally to medical devices and examples of methods of use.

Embodiments of the invention include devices for performing thrombectomy or embolectomy in the internal carotid artery and other vessels of a patient.

### BACKGROUND

Acute Ischemic Stroke (AIS) can be caused by thrombus, embolus or other occlusions in regions of the internal carotid artery (ICA) such as the Petrous part, Cavernous part or Cerebral part. Approaches for performing thrombectomy or embolectomy to treat AIS include positioning a balloon guiding catheter in the carotid artery at a location upstream from the occlusion, typically at a proximal location in the artery such as the Cervical part. After the balloon is inflated to provide antegrade blood flow cessation, suction can be applied to the catheter to retrieve the embolus. Thrombectomy tools such as stent retrievers can also be delivered directly to the embolus through the guiding catheter to break up the embolus and enhance the retrieval process.

These thrombectomy procedures may involve placing a sheath through an arteriotomy in the patient's common femoral artery, and delivering the guiding catheter to the ICA through the sheath. For example, an 8-9 French (Fr) inner diameter (ID) (0.015-0.118 inches) sheath having a length on the order of twenty-five centimeters can be used to provide the access to the arterial tree through the arteriotomy. A balloon guiding catheter having a 7-8 Fr outer diameter (OD) (0.092-0.105 inches), commonly about ninety centimeters in length, can then be delivered to the ICA through the sheath. A 10-11 Fr (0.131-0.144 inch) arteriotomy may be required for the sheath during procedures of these types. Unfortunately these relatively large arteriotomies can enhance the risk of bleeding, especially since patient's undergoing these procedures may be receiving thrombolytics that may increase the risks of hemorrhagic complications. (1 inch converts to 2.54 cm and 1 Fr/French convert to 0.033 cm)

Relatively small diameter distal access aspiration catheters (e.g., up to about 0.087 inch OD) are sometimes used during thrombectomy in the ICA. Such distal aspiration catheters include the ACE 68 from Penumbra, Inc. and the Sophia Plus from Microvention, Inc. For example, during these procedures the distal aspiration catheter can inserted with the end positioned at the distal middle cerebral artery. Other thrombecotomy tools such as stent retrievers are sometimes delivered to the intracranial vasculature through distal access catheters used in this manner. However, balloon guiding catheters have IDs that are too small to accommodate these distal aspiration catheters. Other known balloon guide catheters include the MO.MA Ultra and Cello devices from Medtronic, Inc., and the Flowgate2 device from Stryker Neurovascular. The relatively long period of time required to place a sheath and then a balloon guide catheter can detract from the benefits of this treatment.

Stents and other endovascular tools are sometimes placed in the ICA or other vasculature using guiding sheaths that do not have balloons. Guiding sheaths are typically about ninety centimeters in length. These devices act as a combination of access sheath and guiding catheter. The need for a separate sheath is obviated by the use of these guiding sheaths since they are sufficiently long to provide access to the target vessel. Although guiding sheaths do not provide arterial occlusion, they can be rapidly placed.

There is a continuing need for improved devices and methods for performing mechanical revascularization such as thrombectomy and embolectomy in the ICA and other vasculature. In particular, there is a need for such devices and methods that provide enhanced efficacy. Devices and methods of these types that can improve the efficiency of health care delivery would be especially desirable.

US2014107575A1 discloses a balloon catheter having at least two lumens. One of the lumens is said to be a large working lumen. The balloon catheter is intended to be useful as a guide catheter or microcatheter and used in a variety of therapeutic and diagnostic procedures. In particular, it is said to have value in treating neurovascular embolic strokes in combination with other devices which are delivered to the stroke site through that working lumen. The remainder of the lumens typically are used to inflate and to deflate the balloon.

US2003023204A1 discloses methods and apparatus that are intended for removing emboli during an angioplasty, stenting or surgical procedure comprising a catheter having a funnel-shaped occlusion balloon of uniform thickness disposed on a distal end of the catheter. The occlusion balloon is said to be fused to the distal end and it is intended to provide a substantially seamless flow transition into a working lumen of the catheter. Additionally, a distal edge of the occlusion balloon is configured to be in close proximity with an inner wall of a vessel to facilitate blood flow into the catheter and efficiently remove emboli.

WO2010017537A2 discloses an apparatus that is intended for accessing and/or removing material from a body lumen. The apparatus is said to include a tubular sheath including a shaft having a proximal end and an expandable distal end that is expandable between a collapsed configuration and an expanded, tapered configuration. In one aspect, the expandable member is said to include inner and outer membranes attached together and to the shaft. Optionally, the inner membrane is said to include a reinforcement layer and/or the outer membrane may have a longer chord length than the inner membrane, to enhance the expandable member expanding such that a distal opening is defined by the expandable member having s a relatively large diameter tapering proximally to a lumen of the shaft. Optionally, the apparatus is said to include a dilator and/or one or more treatment devices receivable in the lumen of the shaft.

US2012116350A1 discloses systems and methods that are intended for delivering implantable devices, catheters, or substances in or near and/or restoring flow through body lumens, such as blood vessel lumens are described. A catheter is said to have a proximal portion of a first diameter and a distal portion of a second diameter (smaller than the first diameter) is advanced into a body lumen. The distal portion of the catheter is said to be caused to expand to a diameter that is larger than the second diameter but no larger than the first diameter. A working device is then said to be advanced out of the distal end of the catheter and used to remove obstructive matter, deliver an implantable device or substance and/or restore flow. The distal portion can be reduced in diameter prior to removal from the body. A stand alone, guide catheter is also disclosed that is said to possess high resistance to kinking even with a very thin wall.

WO0191844A1 discloses apparatus and methods intended for effective removal of emboli or harmful fluids during vascular procedures, such as angiography, balloon angioplasty, stent deployment, laser angioplasty, atherectomy, intravascular ultrasonography and other therapeutic and diagnostic procedures. A catheter with an occluder mounted at its distal end is a said to create an occlusion proximal to the lesion. The catheter is said to provide a pathway for introducing a treatment catheter. Prior to, during or subsequent to the procedure, suction is activated to establish retrograde flow to remove emboli from the site. Additionally, a thin catheter with a distal fluid ejection nozzle is said to be introduced distal to the treatment site to rinse emboli from the treatment site. The suction flow and/or ejected fluid flow may be varied in a pulsatile manner to simulate regular blood flow and/or perturb settled emboli into being captured. The method establishes a protective environment before any devices enter the site to be treated.

WO03061457A2 discloses methods and devices that are intended for performing percutaneous and surgical interventions. The devices are said to comprise a tubular portion and retractable mechanism at the distal end of the tubular portion. The retractable mechanism is intended to prevent the device from pulling out of an anatomical structure during complex interventions, for example, when switching from an antegrade to a retrograde approach within a blood vessel, enables the use of a single sheath when declotting AV hemodialysis fistulas and can provide occlusion of blood flow during interventions and means of removal of debris or clot from the blood vessel.

WO2014204860A1 discloses an arterial access device that is said to have an internal lumen and a proximal port, the arterial access device is said to be sized and shaped to be inserted directly into an arterial access site in the common carotid artery such that the lumen provides a passageway for an interventional device to be inserted via the proximal port into the carotid artery. The arterial access device is said to have a distal portion that is configured to be inserted into an arterial pathway through the access site, and a proximal portion configured to extend outward from the access site when the distal portion is in the arterial pathway.

### SUMMARY

The invention is defined by independent claim 1, with further embodiments defined in the dependent claims. Embodiments of the invention include tools for performing thrombectomy, embolectomy and other procedures in patients' internal carotid arteries and other vasculature. A balloon guiding sheath in accordance with embodiments comprises an elongated sheath having a working section length of at least ninety centimeters, a proximal end portion and a distal end portion. The sheath has an access port is on the proximal end portion and a distal port on the distal end portion. A working lumen extends through the sheath between the access port and the distal port. An inflatable balloon is on the distal end portion of the sheath. An inflation port is on the proximal end portion of the sheath. An inflation lumen extends in the sheath between the inflation port and the balloon. The sheath is configured to enable direct insertion of the working length of the sheath into a patient's vasculature through an arteriotomy to position the balloon at a target site. The working lumen comprises an inner layer, an intermediate layer and an outer layer, and the intermediate layer is formed from coiled or braided strands of material, wherein one or more of the material properties of the layers, the thicknesses of the layers, the type of the layers and the number of the layers varies along a length of the sheath to provide graded longitudinal and rotational stiffness characteristics along the length thereof. Either the inflation lumen is defined in the outer layer, or the inflation lumen is defined as a structure within the working lumen.

The balloon guiding sheath has an outer diameter between about 0.264 and 0.315 cm (about 0.104 and 0.124 inches), and the working lumen has an inner diameter between about 0.221-0.287 cm (about 0.087-0.113 inches). The sheath can include a recess at the distal end portion, with the balloon being located substantially within the recess when the balloon is in an uninflated state. Embodiments of the sheath have a working length sufficiently long to enable the distal end portion to reach a patient's internal carotid artery from a femoral artery arteriotomy.

A method for using the balloon guiding sheath in accordance with embodiments of the disclosure includes inserting the balloon guiding sheath directly into a patient's vasculature through an arteriotomy in the patient's femoral artery. The balloon guiding sheath is advanced through the patient's vasculature and positioned at the distal end portion in the patient's internal carotid artery. The balloon is inflated. Relatively low pressure is applied to the access port to suction an embolus. The balloon is deflated, and the balloon guiding sheath is withdrawn through the arteriotomy in the femoral artery.

### BRIEF DESCRIPTION OF THE DRAWINGS

FIG. 1 is an isometric illustration of a balloon guiding sheath in accordance with embodiments of the invention.
FIG. 2 is a cross sectional illustration of the guiding sheath shown in FIG. 1, taken along line 2 - 2 in FIG. 1.
FIG. 3 is a cross sectional illustration of the guiding sheath shown in FIG. 1, taken along line 3 - 3 in FIG 1.
FIG. 4A is detailed cross sectional illustration of the distal end portion of the guiding sheath shown in FIG. 1, taken along ling 4A - 4A in FIG. 1, showing the balloon in an uninflated state.
FIG. 4B is a detailed cross section illustration of the distal end portion of the guiding sheath shown in FIG. 4A, showing the balloon in an inflated state.
FIG. 5 is an illustration of a portion of an internal carotid artery (ICA) into which a guiding sheath of the invention has been positioned during an exemplary thrombectomy procedure.
FIG. 6A is a detailed cross sectional illustration of the distal end portion of a guiding sheath in accordance with other embodiments of the invention, showing the balloon in an uninflated state.
FIG. 6B is a detailed cross section illustration of the distal end portion of the guiding sheath shown in FIG. 6A, showing the balloon in an inflated state.
FIG. 7 is an isometric illustration of an endovascular instrument set in accordance with embodiments of the disclosure.
FIG. 8 is an illustration of the distal end portion of the expansion tool shown in FIG. 7, with the balloon in the expanded state.
FIG. 9 is an illustration of the distal end of the expansion tool shown in FIG. 7 located in the sheath/guide catheter shown in FIG. 7, with the balloon of the expansion tool inflated to diametrically expand the distal end portion of the sheath/guide catheter.
FIG. 10 is an illustration of a distal end portion of an expansion tool in accordance with other embodiments of the disclosure, shown located in a sheath/guide catheter, and with the balloon of the expansion tool inflated to diametrically expand the distal end portion of the sheath/guide catheter.
FIG. 11 is an isometric illustration of an intermediate access aspiration catheter in accordance with embodiments of the disclosure.
FIG. 12 is an illustration of the intermediate access aspiration catheter shown in FIG. 11 following its insertion into the ICA of a patient.
FIG. 13 is an illustration of an intermediate access aspiration catheter having a tapered tip in accordance with other embodiments of the disclosure.
FIG. 14 is an illustration of the intermediate access aspiration catheter shown in FIG. 13 following its insertion into the ICA of a patient.
FIG. 15 is an illustration of a stent retriever in accordance with embodiments of the disclosure.
FIG 16 is a cross sectional illustration of the stent retriever shown in FIG. 15, taken along line 16 - 16 in FIG. 15.
FIG. 17 is an illustration of a stent retriever in accordance with other embodiments of the disclosure.

### DETAILED DESCRIPTION

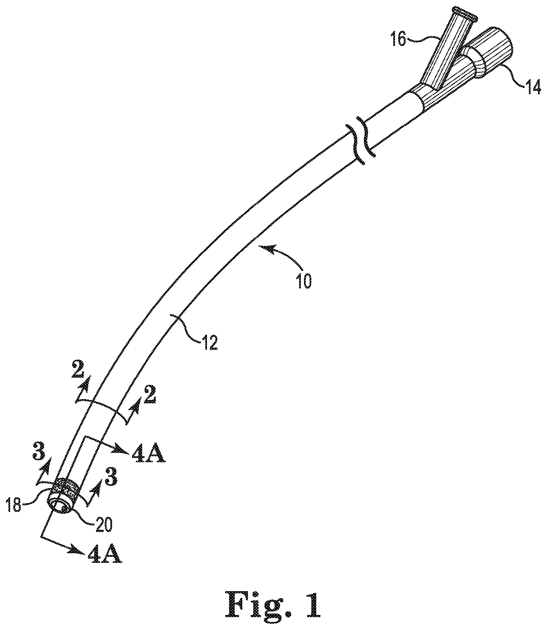

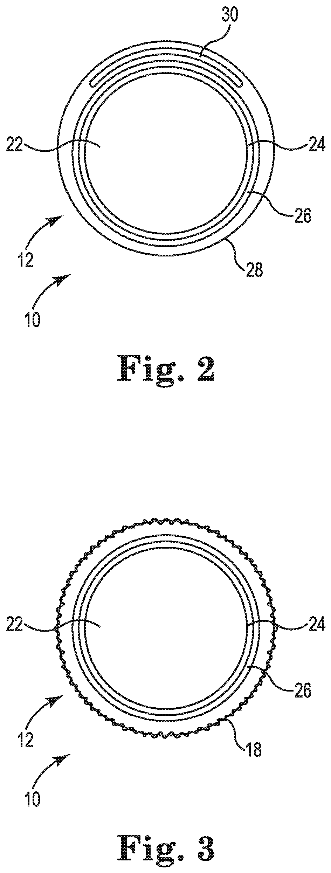

A balloon guiding sheath 10 in accordance with embodiments of the invention can be described generally with reference to FIG. 1. As shown, guiding sheath 10 includes an elongated sheath 12 having an access port 14 and inflation port 16 on its proximal end portion, and a balloon 18 and port 20 on the its distal end portion. FIG. 2 is a cross sectional view of an embodiment of the sheath 12 at a location between the access port 14 and balloon 18. The sheath 12 is a tubular structure that includes a primary or working lumen 22 extending between the access port 14 and port 20. Lumen 22 is defined by an inner layer 24, intermediate layer 26 and an outer layer 28 in the illustrated embodiment. The inner layer 24 can be formed from polymer such as PTFE (polytetrafluoroethylene). The intermediate layer 26 is formed from coiled or braided strands of material, material can be stainless steel or polymer wire. The outer layer 28 can be formed from polymer material such as nylon. An inflation lumen 30, shown in the outer layer 28 in the illustrated embodiment, extends between the inflation port 16 and the balloon 18. In other examples (not shown) the inflation lumen is in other structures of the sheath 12, or the inflation lumen is a tubular structure within the lumen 22, in accordance with the claimed invention. Other examples (now shown) include more than one inflation lumen, and/or such lumens having different cross sectional shapes.

The primary structural components (e.g., layers 24, 26 and 28) of the sheath 12 are configured to provide the sheath with longitudinal and rotational stiffness characteristics (e.g., the capabilities of being able to be pushed and/or twisted between its proximal and distal ends) to enable the device to be relatively rapidly delivered to the desired or target location in the patient's vasculature. In examples (not shown), the sheath 12 can be formed from more or fewer and/or different types of structural layers. For example, a lubricious coating layer can be applied to all or portions of the exterior surface of the outer layer 28. Other examples do not have an outer friction-reducing coating. The outer layer 28 of the sheath 12 can be formed of low-friction material in examples. The composition of the sheath 12 along its length is varied to provide graded longitudinal and rotational stiffness characteristics. These graded stiffness characteristics is provided, for example, by varying the material properties and/or thicknesses of the layers such as 24, 26 and 28 along the length of the sheath 12, and/or by including more or fewer and/or different such structural layers. The guiding sheath 10 is sufficiently long to enable the device to be advanced from the arteriotomy through the vasculature, and the distal end portion and port 20 positioned at the target site. In embodiments of the invention, the sheath has a working length that can be inserted into the patient of at least ninety centimeters. In examples, the guiding sheath can be longer or shorter.

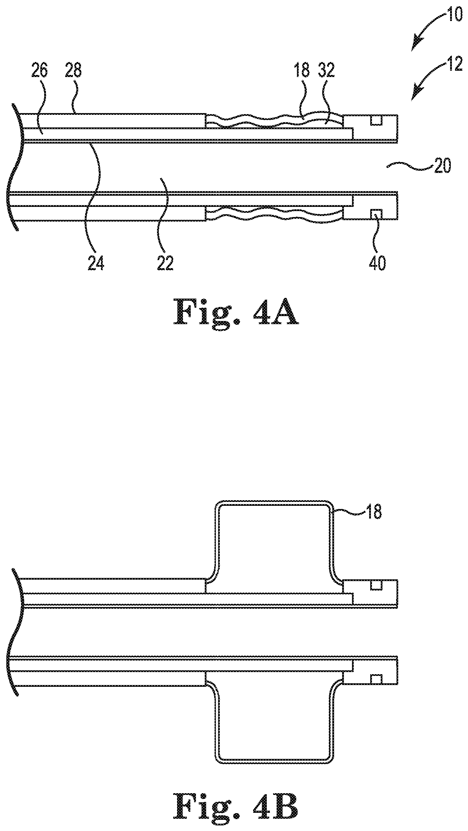

The distal end portion of the sheath 12 including the balloon 18 can be described with reference to FIGs. 3, 4A and 4B. Balloon 18 is located in a recess 32 on the distal end portion, adjacent the port 20. In the illustrated embodiment the recess 32 is in the outer layer 28, but in other embodiments the recess is located in other or additional layers of the sheath 12. The recess 32 or other structure opening into the interior of the balloon 18 is coupled to the inflation lumen 30 so inflation fluid can be delivered to and withdrawn from the balloon through the inflation port 16. FIG. 4A shows the balloon 18 in the wrapped, undeployed and uninflated state. In the illustrated embodiment the uninflated balloon 18 is a low profile structure that has a diameter that is not, or not substantially, greater than the diameter of the adjacent portions of the sheath 12 to optimize the ability of the guiding sheath 10 to be inserted. In other embodiments (not shown), the balloon 18 can be mounted over the outer surface of the sheath. FIG. 4B shows the balloon in inflated, deployed and diametrically expanded state. Balloon 18 is sized and configured to occlude arteries or other vessels in which it is positioned. In embodiments, for example, the balloon 18 can be expanded to about ten millimeters in diameter, and can be about ten millimeters in length. The balloon 18 can also be rewrapped or deflated tight enough through the withdrawal of fluid through lumen 30 to optimize the ability of the guiding sheath 12 to be withdrawn.

Embodiments of sheath 12 also include a radiopaque marker 40. Marker 40 is shown on distal end portion of the sheath 12 in the embodiments of FIGs. 4A and 4B. Marker 40 can be imaged during procedures to identify the location of the sheath 12, and in particular the distal end of the sheath, in the patient's vasculature.

Guiding sheath 10 has an outer diameter (OD) of about about 0.264-0.315 cm (0.104-0.124 inches). Devices with these ODs can have inner diameters (IDs), for example, in the range of 0.221-0.287 cm [0.087-0.113 inches]). A particularly useful embodiment of guiding sheath 10 has an OD of less than 0.27 cm (0.105 inch) (e.g., about 9.2 Fr) and an ID of greater than 0.22 cm (0.087 inch) (e.g., about 6 Fr). In embodiments, the sheath 12 has a generally constant diameter. In other embodiments the sheath 12 has a generally increasing diameter along its length in the direction from the distal end portion toward the proximal end portion, so that portions of the sheath entering the arteriotomy during the insertion of the guiding catheter 10 to the target site will not generally decrease in size, thereby minimizing bleeding at the arteriotomy. Any transitions along the outer surface of the sheath 12 are preferably smooth. (1 inch converts to 2.54 cm and 1 Fr/French convert to 0.033 cm)

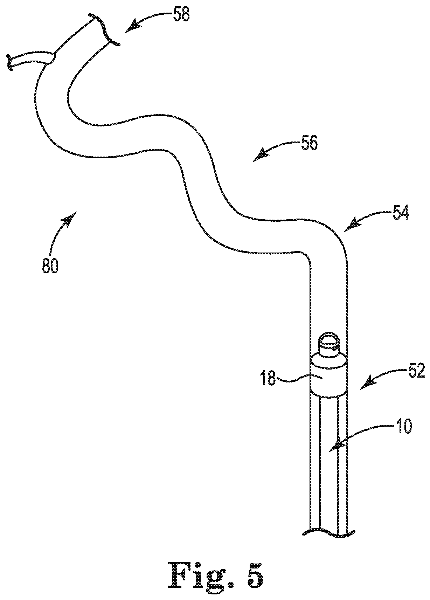

The exemplary use of a guiding sheath 10 of the invention to perform a thrombectomy or embolectomy can be described with reference to FIG. 5, which shows a portion of an internal carotid artery (ICA) 50 including the Cervical part 52, Petrous part 54, Cavernous part 56 and Cerebral part 58. Guiding sheath 10 combines the functionality of a sheath and a balloon guide catheter. The guiding sheath 10 can be inserted directly (e.g., without the use of a sheath or other introducer) through an arteriotomy (e.g., in the femoral artery), and advanced through the patient's vasculature with the balloon 18 in its uninflated state. The surgeon can continue this insertion procedure to position the distal end portion and port 20 at the target site, upstream from the embolus to be removed. Fluid is then applied through the inflation port 16 to deploy or inflate the balloon 18 and occlude the ICA. By applying relatively low pressure or a vacuum to the access port 14, embolus downstream from the port 20 can be removed by suction. In embodiments such as that shown in FIG. 5, the distal end portion of the guiding sheath 10 is positioned with the balloon 18 and port 20 in the Cervical part 52 of the ICA (i.e., at a relatively proximal location in the ICA), enabling the removal of embolus (not shown) in the Petrous part 54, Cavernous part 56 and Cerebral part 58. In embodiments, guiding sheaths 10 having an OD between about 6 and 9 Fr can be efficaciously placed in the Cervical part 52 of many patients. In other embodiments, guiding sheaths 10 having these or smaller ODs such as 7-8 Fr can be inserted further (i.e., distally) into the ICA (e.g., to the Petrous part 54 or beyond). Imaging, using the marker 40, can be used by the surgeon during insertion of the guiding sheath 10 to position the end portion and port 20 at the desired location in the ICA. Following the removal of the embolus, the fluid can be removed through the inflation port 16 to uninflate the balloon. The guiding sheath 10 can then be withdrawn from the patient's vasculature. Methods of use are not part of the claimed invention.

In other embodiments, additional endovascular tools such as distal access aspiration or mechanical stent retriever tools can be used with guiding sheaths 10 that are positioned in accordance with methods described above. During these procedures, the additional endovascular tools can be inserted into the access port 14, fed through the lumen 22 and port 20, and delivered to the target site.

Guiding sheath 10 offers a number of important advantages. For example, the ability to place one device instead of two (i.e., a standard sheath and guide catheter), or a single device in connection with certain procedures such as the thrombectomy procedure described above, shortens the procedure time and reduces the number of instruments used. Smaller arteriotomies can also be used since the diameters of the devices used for a given procedure are reduced. This advantage is particularly important in connection with thrombectomy procedures because patients undergoing procedures of these types may be receiving thrombolytics that can increase the risk of bleeding at the arteriotomy. Morbidity associated with embolus fragmentation can be reduced by the temporary antegrade flow cessation. The relatively large IDs of the devices markedly improve suction efficiency by providing flow rates that can be up to two or three, or even more times those of current devices. The stiffnesses and other characteristics of the sheath 12 can provide improved support for distal access devices and prevent "back out" that can result in loss of access during use. The relatively large balloon inflation lumens provided by the device enable the balloon to be rapidly deployed and uninflated, thereby reducing the time required for procedures. The efficiency and efficacy of healthcare services are thereby enhanced. Because of their enhanced size features, embodiments of guiding sheaths 10 are free from or do not have hydrophylic or other friction-reducing coatings that might be susceptible to separation from the guiding sheath during use.

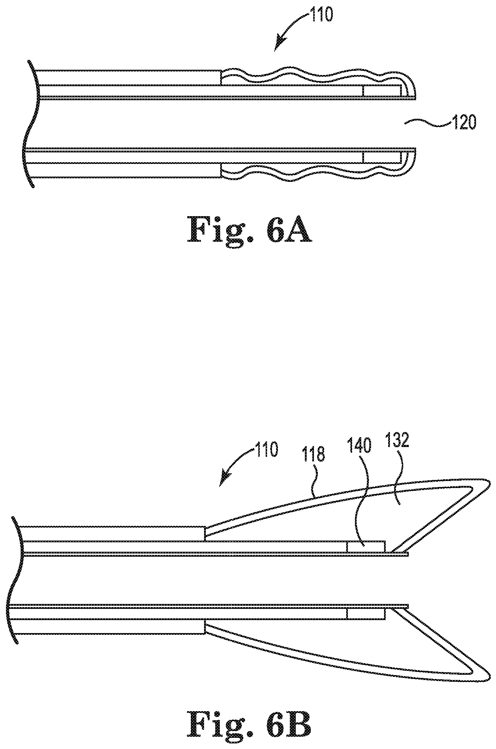

FIGs. 6A and 6B illustrate the distal end portions of a guiding sheath 110 in accordance with embodiments of the invention. As shown in FIG. 6B, when the balloon 118 is in the deployed or expanded shape, it has a concave or funnel-shaped opening that in the illustrated embodiment extends distally from the distal ends of the inner, intermediate and outer layers 24, 26 and 28. In other embodiments the distal end of the inflated balloon 118 extends distally of one or more of the layers such as 24, 26 and 28 of the sheath 112. During use of the guiding sheath 110, the asymmetric and concave shape of the inflated balloon 118 can cause the retrieved thrombus to be preferentially drawn to the port 120, thereby reducing the possibility of the thrombus being trapped between the distal end of the guiding sheath and the vessel wall. By enhancing the collection of the thrombus, guiding sheath 110 can provide improved treatment efficacy. Other than the differences shown in FIGs. 6A and 6B and described above, guiding sheath 110 can be similar to or substantially the same as guiding sheath 10.

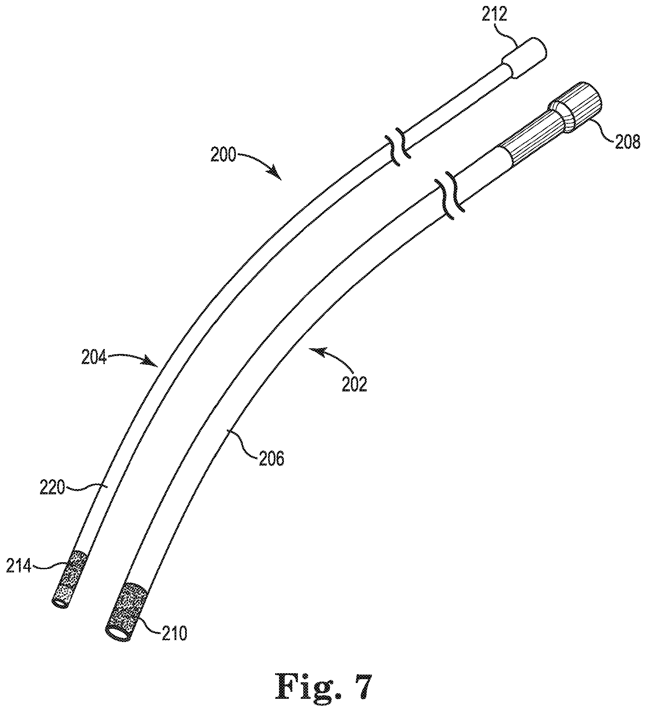

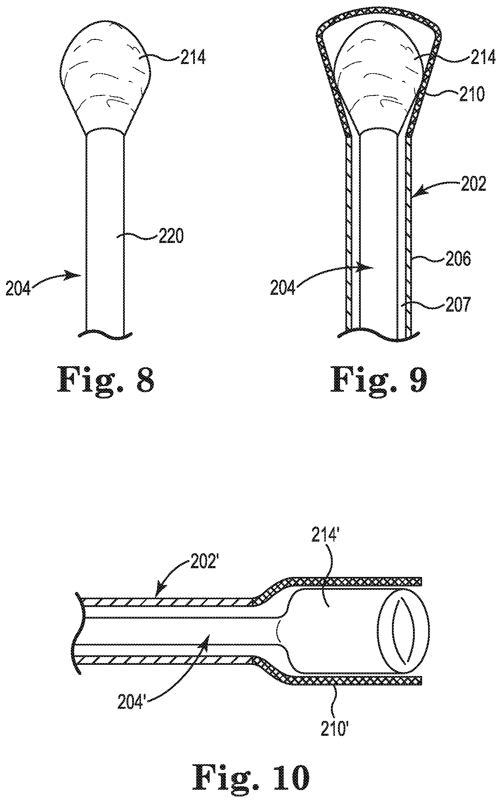

FIG. 7 is an illustration of an endovascular instrument set 200 in accordance with embodiments ,not forming part of the claimed invention, that includes a sheath/guide catheter 202 and an expansion tool 204. Sheath/guide catheter 202 includes an elongated tubular member 206 having an access port 208 on its proximal end and a diametrically expandable tip 210 with a distal port on its distal end. The tubular member 206 has a lumen 207 (e.g., FIG. 9), and can be a structure that is the same as or similar to that of the sheath 12 described above in connection with FIGs. 1-3, or other known or conventional sheaths or guide catheters. The structure of the tubular member 206 will be configured to enable the sheath/guide catheter 202 to be inserted into and through a patient's vasculature, and to position the tip 210 at a target site while the tip is in a retracted or diametrically-reduced state. The tip 210 is diametrically expandable in response to a force applied from the inside of the tip, and capable of retaining its expanded shape. In embodiments, for example, the tip 210 can be formed from malleable metals or other materials. In other embodiments the tip 210 can be an expandable braided or coiled wire structure. In still other embodiments the tip 210 is formed from a plurality of petal members that open like a flower. Shape memory materials such as nitinol can be used in these and other embodiments of the tip 210.

Expansion tool 204 includes an elongated tubular member 220 having an inflation port 212 on its proximal end and a diametrically expandable balloon 214 on its distal end. A lumen (not shown) extends through the tubular member 220 between the inflation port 212 and the balloon 214. The outer diameter of the tubular member 220 is less than the inner diameter of the lumen 207 of the sheath/guide catheter 202. The balloon 214 is shown in its uninflated or diametrically retracted state in FIG. 7. As shown in FIG. 8, the balloon 214 can be inflated and diametrically expanded by the application of fluid though the inflation port 212.

The use of instrument set 200 can be described in connection with FIGs. 7-9. **In** embodiments, the sheath/guide catheter 202 is used to remove embolus from ICAs in a manner similar to that described above. Following the insertion of sheath/guide catheter 202 and the positioning of its tip 210 (in the retracted state) at the target site, as described above, the expansion tool 204 is inserted through the sheath/guide catheter with the balloon 214 in the uninflated state to position the balloon adjacent the sheath/guide catheter tip. The balloon 214 is then deployed or inflated and diametrically expanded. The expansion of the balloon 214 applies sufficient forces on the tip 210 of the sheath/guide catheter 202 to cause the tip to take its expanded diameter shape (e.g., a concave shape, as shown in FIG. 9). By this expansion the tip 210 can be urged into contact with the inner walls of the vessel. The balloon 214 can then be returned to its uninflated state by withdrawing the inflation fluid through the port 212, and the expansion tool 204 withdrawn from the sheath/guide catheter 202. The tip 210 will remain in the expanded diameter state following the withdrawal of the expansion tool 204. The sheath/guide catheter 202 can then be used in connection with an endovascular procedure. For example, suction can be applied to the access port 208 to remove embolus. Alternatively, a tool such as a distal access or other catheter or a stent removal tool can be inserted through the sheath/guide catheter 202 and delivered to the target site. Following the procedure, the sheath/guide catheter 202 is withdrawn from the patient. The tip 210 is sufficiently resilient that it will diametrically retract or collapse during the removal. In other embodiments the tip 210 is configured to be retracted by the surgeon before removal of the sheath/guide catheter 202.

In embodiments, the tip 210 and/or balloon 214 can be configured to take other diametrically expanded shapes. FIG. 10, for example, illustrates a sheath/guide catheter 202' having a tip 210' and an expansion tool 204' with a balloon 214' that are configured and cooperate to symmetrically expand the tip of the sheath/guide catheter. Other features of the sheath/guide catheter 202' and expansion tool 204' can be the same as or similar to those of sheath/guide catheter 202 and expansion tool 204 described above. Similarly, the sheath/guide catheter and/or expansion tool can be configured to cooperate to provide tips that have other shapes when in the diametrically expanded state.

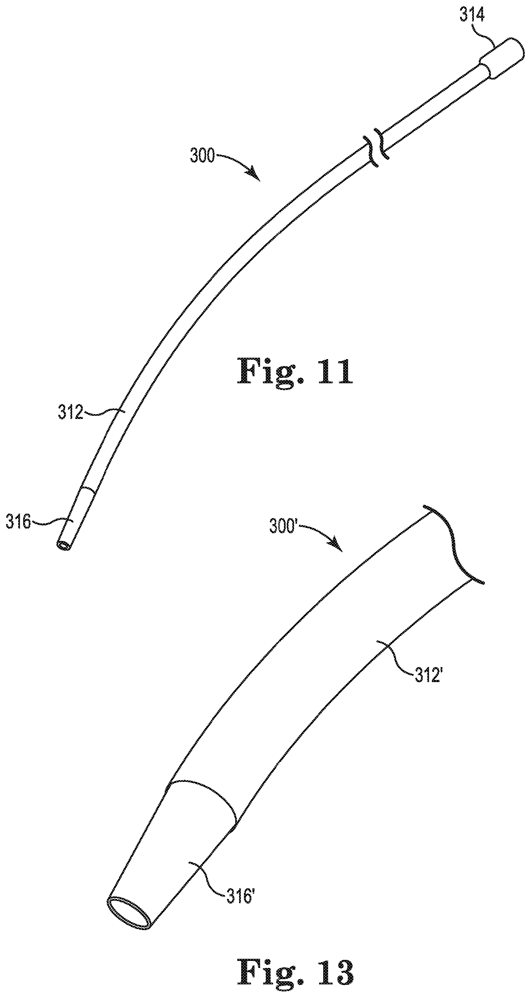

FIG. 11 is an illustration of an intermediate access aspiration catheter 300 in accordance with embodiments of the disclosure, not forming part of the claimed invention. As shown, catheter 300 includes an elongated tubular member 312 having an access port 314 on its proximal end portion, and a tip 316 on its distal end portion. Intermediate aspiration catheter 300 is configured to be inserted into intermediate locations of a patient's ICA. In embodiments, for example, the catheter 300 is configured to have its tip 316 positioned in the Petrous part or Cavernous part of the ICA. One embodiment of the intermediate aspiration catheter 300 has an OD of about 8 Fr (e.g., about 0.27 cm [0.105 inch]). The ID of such an 8 Fr catheter 300 can be between about 0.20-0.23 cm (0.080-0.090 inches), in embodiments. Other embodiments of the catheter 300 have an OD between about 7 Fr and 8 Fr (e.g., about 0.23-0.27 cm [0.105-0.118 inches]). The IDs of such 8-9 Fr catheters 300 can range between about 0.20-0.23 cm (0.080-0.090 inches). The catheter 300 can be about ninety centimetres long in embodiments. Other embodiments can have other lengths, which can depend on factors such as the applications for which the device is used.

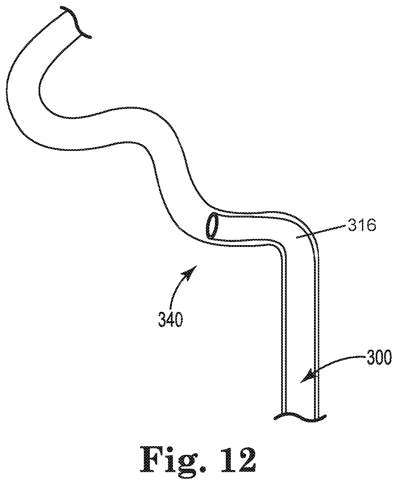

Tip 316, which can be between ten and twenty centimeters in length in embodiments (other embodiments have other lengths), is more flexible that the portions of the tubular member 312 between the tip and access port 314. In embodiments, the tip 316 is sufficiently long and flexible to enable the tip to be positioned in the Petrous part or Cavernous part of the ICA. FIG. 12, for example, shows the catheter 300 located with the tip 316 in the Petrous part 340 of an ICA. The portions of tubular member 312 proximal to the tip 312 are sufficiently stiff that they can be manipulated during the insertion of the catheter 300 to relatively quickly position the tip 312 at the desired location. In embodiments, the tubular member 312 can be the same as or similar to the structure of the sheath 12 of the guiding sheath 10 described above. In other embodiments the tubular member 312 has other structures that provide the functional characteristics described above. For example, the tip 316 can have fewer component layers than that of the guiding sheath 10 to provide the appropriate flexibility of the tip.

In embodiments, the catheter 300 can be inserted directly through an arteriotomy and advanced to a target site. In other embodiments, the catheter 300 is used with other devices such as the guiding sheath 10 (i.e., the catheter is advanced to the target site though the guiding sheath after the guiding sheath has been inserted and positioned). After the catheter 300 is inserted and the tip 316 positioned (i.e., adjacent or upstream of an embolus), suction can be applied to the device through the access port 314 to collect the embolus.

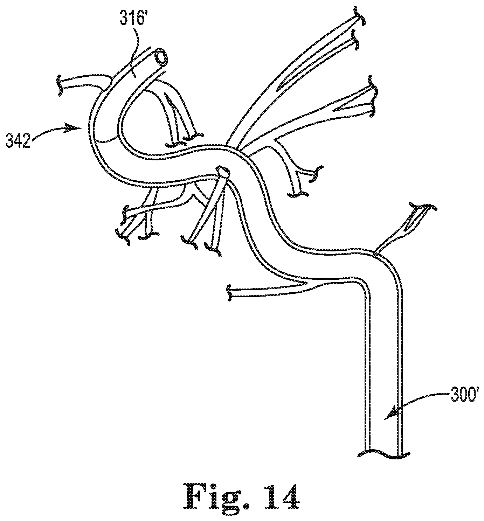

FIG. 13 is an illustration of the distal end portion of another intermediate access aspiration catheter 300' in accordance with embodiments of the disclosure, not forming part of the claimed invention. As shown, catheter 300' has a tapered tip 316' that tapers to a smaller diameter than the diameter of the tubular member 312' . Other than the tapered shape of the tip 316', features of catheter 300' can be the same as or similar to those of catheter 300 described above in connection with FIG. 11.
Embodiments of catheter 300' are configured to have its tip 316' positioned in the Cavernous part or Cerebral part of the ICA, for example adjacent or in the Middle Cerebral artery. FIG 14, for example, shows the catheter 300' located with the tip 316' in the Cerebral part 342 of the ICA. In embodiments, catheter 300' has a tubular member with an OD of about 8 Fr and a tip 316' that tapers to an OD of about 6-7 Fr. The ID of the distal end of the tip 316' in such embodiments can be, for example, in the range of 0.19-0.22 cm (0.075-0.085 inches). Other embodiments of the catheter 300' have an OD between about 7 Fr and 8 Fr (e.g., about 0.23-0.27 cm [0.092-0.105 inches]), and a tip 316' that tapers to an OD of about 0.080-0.090 Fr. Yet other embodiments of the catheter 300' have an OD between about 8 Fr and 9 Fr (e.g., about 0.27-0.30 cm [0.105-0.118 inches]), and a tip 316' that tapers to an OD of about 7.5-8.5 Fr. Catheter 300' can be inserted, advanced and used in manners similar to or the same as those described above in connection with catheter 300, including in connection with a guiding sheath 10.

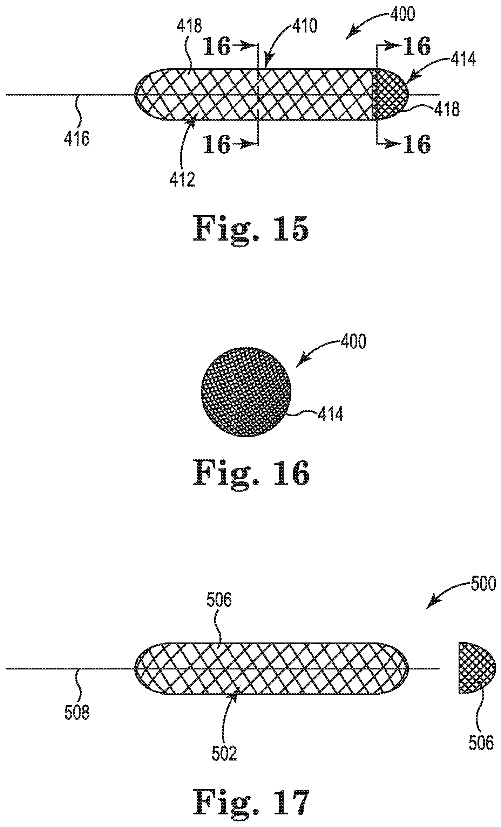

FIGs. 15 and 16 are illustrations of a stent retriever 400 in accordance with embodiments of the disclosure, not forming part of the claimed invention. Stent retriever 400, which is shown in its diametrically expanded or deployed state in FIG. 15, includes a body 410 having a proximal or first portion 412 and a distal tip 414. A wire 416 extends from the body 410. Both the first portion 412 and the distal tip 414 are formed from a mesh of wires 418. In embodiments, the wires 418 are metal such as stainless steel or nitinol, and can be braided. As shown, the mesh density of the wires 418 in the distal tip 414 is greater than the mesh density of the wires in the first portion 412 (i.e., the first portion is more porous than the distal tip). In embodiments, the mesh density of the wires 418 in the tip 414 is sufficiently high to cause the tip to be substantially closed (i.e., non-porous). In embodiments, the distal tip 414 can be approximately one-fourth to one-third of the length of the body 410, although it has other lengths in other embodiments. As described in greater detail below, the tip 414 is configured to trap portions of emboli that are released and not trapped by the first portion 412 during thrombectomy procedures. In embodiments, the mesh density of the wires 418 in the first portion 412 is sufficiently low to enable the wires of the first portion to engage an embolus during thrombectomy procedures. In embodiments, the stent retriever 400 is configured as a self-expandable device. In other embodiments the stent retriever 400 is configured as a balloon-expandable device.

Stent retriever 400 is placed in a reduced-diameter or unexpanded state (not shown) for delivery. Conventional or otherwise known structures (e.g., a removable sheath; not shown) can be used to place the stent in the unexpanded state. While in the unexpanded state, the stent retriever 400 is inserted through the patient's vasculature to the target site in a conventional or otherwise known manner. For example, in embodiments the stent retriever 400 can be delivered though a guiding sheath 10 of the type described above. At the target site the stent retriever 400 is pushed through the embolus. In embodiments, at least the tip 414 is positioned beyond the embolus. After the stent retriever 400 is positioned, it is actuated or deployed to diametrically expand (e.g., by unsheathing a self-expanding embodiment). With the stent retriever 400 in its expanded state, the surgeon can manipulate the device, including the first portion 412, to engage the embolus and release the embolus from the vessel. The stent retriever 400 and embolus are then withdrawn from the vessel. In embodiments, much if not all of the embolus will be engaged by the relatively low density wire mesh of the first portion 412. Portions of the embolus that break free or are otherwise not engaged by the first portion 412 can be captured by the tip 414 during the withdrawal of the stent retriever 400. Stent retriever 400 thereby provides enhanced efficacy by allowing for less thromboembolic events.

FIG. 17 is an illustration of a stent retriever 500 in accordance with other embodiments of the disclosure, not forming part of the claimed invention. Stent retriever 500, which is shown in its diametrically expanded or deployed state in FIG. 17, includes a proximal or first stent member 502 and a distal or second stent member 504. The second stent member 504 is spaced from the first stent member 502 (e.g., by about 2-10 millimeters in embodiments). In other embodiments (not shown) the second stent member 504 is positioned immediately adjacent the first stent member 502. Both the first member 502 and the second member 504 are formed from a mesh of wires 506. In embodiments, the wires 506 are metal such as stainless steel or nitinol, and can be braided. In the illustrated embodiment the first stent member 502 is closed at both ends, and has a first, relatively low mesh density. In embodiments, the mesh density of the wires 506 of the first stent member 502 is sufficiently low to enable the wires to engage an embolus during thrombectomy procedures. The second stent member 504 is located distally of the first member 502 and has a relatively high mesh density of the wires 506 that is greater than the mesh density of the first stent member 502. In embodiments, the mesh density of the wires 506 in the second stent member 504 is sufficiently high to cause the member to be substantially closed. The second stent member 504 is concave, with the opening facing the first stent member 502, in embodiments. As described in greater detail below, the second stent member 504 is configured to trap portions of emboli that are released and not engaged by the first stent member 502 during thrombectomy procedures. In embodiments, the stent retriever 500 is configured as a self- expandable device. In other embodiments the stent retriever 500 is configured as a balloon- expandable device.

Stent retriever 500 is placed in a reduced-diameter or unexpanded state (not shown) for delivery. Conventional or otherwise known structures (e.g., a removable sheath; not shown) can be used to place both the first and second stent members 502 and 504, respectfully in the unexpanded state. While in the unexpanded state, the stent retriever 500 is inserted through the patient's vasculature to the target site in a conventional or otherwise known manner. For example, in embodiments the stent retriever 500 can be delivered though a guiding sheath 10 of the type described above. At the target site the stent retriever 500 is pushed through the embolus. In embodiments, at least the second stent member 504 is positioned beyond the embolus. After the stent retriever 500 is positioned, it is actuated or deployed to diametrically expand both the first and second stent members 502 and 504 (e.g., by unsheathing a self- expanding embodiment). With the stent retriever 500 in its expanded state, the surgeon can manipulate the tool, including the first stent member 502, to engage the embolus and release the embolus from the vessel. The stent retriever 500 and engaged embolus are then withdrawn from the vessel. In embodiments, much if not all of the embolus will be engaged by the relatively low density wire mesh of the first stent member 502. Portions of the embolus that break free or are otherwise not engaged by the first stent member 502 can be captured by the second stent member 504 during the withdrawal of the stent retriever 500. Stent retriever 500 thereby provides enhanced efficacy by allowing for less thromboembolic events.

Although the invention has been described with reference to preferred embodiments, those of skill in the art will recognize that changes can be made in form and detail without departing from the scope of the invention as claimed. In particular, although described in connection with procedures involving the ICA, the devices and examples of methods can be used in other vasculature of patients. The scope of the invention is defined by the appended claims.

## Claims

1. A balloon guiding sheath, comprising:
an elongated sheath (12) having a working section length of at least ninety centimeters, a proximal end portion and a distal end portion;
an access port (14) on the proximal end portion of the sheath (12);
a distal port (20) on the distal end portion of the sheath;
a working lumen (22) extending through the sheath between the access port (14) and the distal port (20);
an inflatable balloon (18) on the distal end portion of the sheath;
an inflation port (16) on the proximal end portion of the sheath;
an inflation lumen (30) extending in the sheath between the inflation port and the balloon;
wherein the sheath is configured to enable direct insertion of the working length of the sheath into a patient's vasculature through an arteriotomy to position the balloon at a target site; and
wherein the working lumen (22) is defined by an inner layer (24), an intermediate layer (26) and an outer layer (28), and the intermediate layer is formed from coiled or braided strands of material, wherein one or more of the material properties of the layers, the thicknesses of the layers (24,26,28), the type of the layers (24,26,28) and the number of the layers (24,26,28) varies along a length of the sheath (12) to provide graded longitudinal and rotational stiffness characteristics along the length thereof, further wherein either the inflation lumen (30) is defined in the outer layer (28), or the inflation lumen (30) is defined as a structure within the working lumen (22), wherein the sheath (12) has an outer diameter between about 0.264 and 0.315 cm (0.104 and 0.124 inches) and the working lumen (22) has an inner diameter between about 0.221 and 0.287 cm (0.087-0.113 inches).

2. The balloon guiding sheath (12) of claim 1, wherein the sheath (12) has a generally constant outer diameter along its working length.

3. The balloon guiding sheath (12) of claim 2, wherein:
the sheath (12) includes a recess (32) at the distal end portion; and
the balloon (18) is located substantially within the recess (32) when the balloon (18) is in an uninflated state.

4. The balloon guiding sheath (12) of claim 3, wherein the sheath (12) has a working length sufficiently long to enable the distal end portion to reach a patient's internal carotid artery from a femoral artery arteriotomy.

5. The balloon guiding sheath (12) of claim 1, wherein:
the sheath (12) includes a recess at the distal end portion; and
the balloon (18) is located substantially within the recess (32) when the balloon (18) is in an uninflated state.

6. The balloon guiding sheath (12) of claim 1, wherein the balloon (18) extends beyond the distal port and defines a funnel-shaped opening into the distal port when the balloon (18) is in an inflated state.

## Patentansprüche

1. Ballonführungshülle, umfassend:
eine längliche Hülle (12), die eine Arbeitsbereichslänge von mindestens neunzig Zentimetern, einen proximalen Endabschnitt und einen distalen Endabschnitt aufweist;
einen Zugangsanschluss (14) an dem proximalen Endabschnitt der Hülle (12);
einen distalen Anschluss (20) an dem distalen Endabschnitt der Hülle;
ein Arbeitslumen (22), das sich durch die Hülle zwischen dem Zugangsanschluss (14) und dem distalen Anschluss (20) erstreckt;
einen aufblasbaren Ballon (18) an dem distalen Endabschnitt der Hülle;
einen Aufblasanschluss (16) an dem proximalen Endabschnitt der Hülle;
ein Aufblaslumen (30), das sich in der Hülle zwischen dem Aufblasanschluss und dem Ballon erstreckt;
wobei die Hülle konfiguriert ist, um ein direktes Einführen der Arbeitslänge der Hülle in das Gefäßsystem eines Patienten durch eine Arteriotomie zu ermöglichen, um den Ballon an einer Zielstelle zu positionieren; und
wobei das Arbeitslumen (22) durch eine Innenschicht (24), eine Zwischenschicht (26) und eine Außenschicht (28) definiert ist und die Zwischenschicht aus gewickelten oder geflochtenen Materialsträngen ausgebildet ist, wobei eines oder mehrere der Materialeigenschaften der Schichten, der Dicke der Schichten (24, 26, 28), der Art der Schichten (24, 26, 28) und der Anzahl der Schichten (24, 26, 28) entlang einer Länge der Hülle (12) variieren, um abgestufte Längs- und Rotationssteifigkeitscharakteristiken entlang der Länge davon bereitzustellen, wobei ferner entweder das Aufblaslumen (30) in der Außenschicht (28) definiert ist oder das Aufblaslumen (30) als eine Struktur innerhalb des Arbeitslumens (22) definiert ist, wobei die Hülle (12) einen Außendurchmesser zwischen etwa 0,264 und 0,315 cm (0,104 und 0,124 Zoll) aufweist und das Arbeitslumen (22) einen Innendurchmesser zwischen etwa 0,221 und 0,287 cm (0,087-0,113 Zoll) aufweist.

2. Ballonführungshülle (12) nach Anspruch 1, wobei die Hülle (12) entlang ihrer Arbeitslänge einen im Allgemeinen konstanten Außendurchmesser aufweist.

3. Ballonführungshülle (12) nach Anspruch 2, wobei:
die Hülle (12) eine Aussparung (32) an dem distalen Endabschnitt einschließt; und
sich der Ballon (18) im Wesentlichen innerhalb der Aussparung (32) befindet, wenn der Ballon (18) in einem nicht aufgeblasenen Zustand ist.

4. Ballonführungshülle (12) nach Anspruch 3, wobei die Hülle (12) eine Arbeitslänge aufweist, die ausreichend lang ist, um dem distalen Endabschnitt zu ermöglichen, um eine innere Halsschlagader eines Patienten von einer Arteriotomie der Oberschenkelarterie zu erreichen.

5. Ballonführungshülle (12) nach Anspruch 1, wobei:
die Hülle (12) eine Aussparung an dem distalen Endabschnitt einschließt; und
sich der Ballon (18) im Wesentlichen innerhalb der Aussparung (32) befindet, wenn der Ballon (18) in einem nicht aufgeblasenen Zustand ist.

6. Ballonführungshülle (12) nach Anspruch 1, wobei sich der Ballon (18) über den distalen Anschluss hinaus erstreckt und eine trichterförmige Öffnung in den distalen Anschluss hinein definiert, wenn der Ballon (18) in einem aufgeblasenen Zustand ist.

## Revendications

1. Gaine de guidage de ballonnet, comprenant :
une gaine allongée (12) ayant une longueur de section de travail d'au moins quatre-vingt-dix centimètres, une partie d'extrémité proximale et une partie d'extrémité distale ;
un orifice d'accès (14) sur la partie d'extrémité proximale de la gaine (12) ;
un orifice distal (20) sur la partie d'extrémité distale de la gaine ;
une lumière de travail (22) s'étendant à travers la gaine entre l'orifice d'accès (14) et l'orifice distal (20) ;
un ballonnet gonflable (18) sur la partie d'extrémité distale de la gaine ;
un orifice de gonflage (16) sur la partie d'extrémité proximale de la gaine ;
une lumière de gonflage (30) s'étendant dans la gaine entre l'orifice de gonflage et le ballonnet ;
dans laquelle la gaine est conçue pour permettre l'insertion directe de la longueur de travail de la gaine dans le système vasculaire d'un patient par le biais d'une artériotomie afin de positionner le ballonnet sur un site cible ; et
dans laquelle la lumière de travail (22) est définie par une couche interne (24), une couche intermédiaire (26) et une couche externe (28), et la couche intermédiaire est formée de brins de matériau enroulés ou tressés, dans laquelle une ou plusieurs des propriétés matérielles des couches, des épaisseurs des couches (24, 26, 28), du type des couches (24, 26, 28) et du nombre de couches (24, 26, 28) varient le long d'une longueur de la gaine (12) pour fournir des caractéristiques de rigidité longitudinale et de rotation graduées le long de la longueur de celles-ci, dans laquelle la lumière de gonflage (30) est en outre définie dans la couche externe (28), ou la lumière de gonflage (30) est définie comme une structure à l'intérieur de la lumière de travail (22), dans laquelle la gaine (12) a un diamètre externe compris entre environ 0,264 et 0,315 cm (0,104 et 0,124 pouce) et la lumière de travail (22) a un diamètre interne compris entre environ 0,221 et 0,287 cm (0,087 à 0,113 pouce).

2. Gaine de guidage de ballonnet (12) selon la revendication 1, dans laquelle la gaine (12) a un diamètre extérieur généralement constant le long de sa longueur de travail.

3. Gaine de guidage de ballonnet (12) selon la revendication 2, dans laquelle :
la gaine (12) comporte un évidement (32) au niveau de la partie d'extrémité distale ; et
le ballonnet (18) est situé sensiblement à l'intérieur de l'évidement (32) lorsque le ballonnet (18) est dans un état non gonflé.

4. Gaine de guidage de ballonnet (12) selon la revendication 3, dans laquelle la gaine (12) a une longueur utile suffisante pour permettre à la partie d'extrémité distale d'atteindre l'artère carotide interne d'un patient à partir d'une artériotomie d'artère fémorale.

5. Gaine de guidage de ballonnet (12) selon la revendication 1, dans laquelle :
la gaine (12) comporte un évidement au niveau de la partie d'extrémité distale ; et
le ballonnet (18) est situé sensiblement à l'intérieur de l'évidement (32) lorsque le ballonnet (18) est dans un état non gonflé.

6. Gaine de guidage du ballonnet (12) selon la revendication 1, dans laquelle le ballonnet (18) s'étend au-delà de l'orifice distal et définit une ouverture en forme d'entonnoir dans l'orifice distal lorsque le ballonnet (18) est à l'état gonflé.
